# EUROPÄISCHE PATENTANMELDUNG

(11) **EP 0 997 533 A1**
(43) Veröffentlichungstag der Anmeldung: **03.05.2000**
(21) Anmeldenummer: 98120206.2
(22) Anmeldetag: 24.10.1998
(51) Int. Cl.: C12P 17/04, C12P 7/62, C12P 7/42, C12N 1/16

(54) **Verfahren zur Gewinnung von Gamma-Decalacton**

(71) Anmelder: HAARMANN & REIMER GMBH, D-37601 Holzminden (DE)
(72) Erfinder: Rabenhorst, Jürgen Dr., 37671 Höxter (DE); Gatfield, Ian Dr., 37671 Höxter (DE)
(74) Vertreter: Mann, Volker, Dr. (DE)

(57) **Zusammenfassung**

Die vorliegende Erfindung betrifft ein Verfahren zur Herstellung von γ-Decalacton, wobei Yarrowia lipolytica enthaltende Kulturen eingesetzt werden.

## Beschreibung

Die vorliegende Erfindung betrifft ein neues Verfahren zur Herstellung von γ-Decalacton.

γ-Decalacton ist aufgrund seiner organoleptischen Eigenschaften ein wichtiger Aromastoff, der einen fruchtigen, pfirsichartigen Geschmack und Geruch aufweist. Grundsätzlich kann γ-Decalacton aus Früchten gewonnen werden. In diesen ist er jedoch in so geringen Mengen vorhanden, daß er wirtschaftlich mittels Extraktion oder Destillation sich nicht isolieren läßt.

Deshalb gab es in den letzten Jahren zahlreiche Versuche, γ-Decalacton in biotechnischen Verfahren herzustellen. Die meisten Verfahren arbeiten unter Einsatz von verschiedenen Hefen. Hierbei werden entweder Rizinusöl oder der Methylester der daraus isolierten Rizinolsäure mit den Hefen umgesetzt. Die dabei erzielten Ausbeuten schwanken zwischen wenigen Milligramm pro Liter bis zu 9,4 g/l in 75 Stunden (FR 2 734 843).

Das Verfahren hat außerdem den Nachteil, daß mit einem Uracil-anseotrophen Material gearbeitet wird und damit zwei getrennte Schritte zur Biomassebildung und Produktion notwendig sind.

Aufgabe der vorliegenden Erfindung ist es daher, ein Verfahren zur Verfügung stellen, daß höhere Ausbeuten ermöglicht.

Diese Aufgabe wird dadurch gelöst, daß eine Yarrowia lipolytica enthaltende Kultur eingesetzt wird.

Es ist erfindungsgemäß möglich, Yarrowia lipolytica im Gemisch mit weiteren Mikroorganismen zu verwenden. Vorzugsweise wird Yarrowia lipolytica jedoch als Reinkultur eingesetzt. Besonders bevorzugt ist es erfindungsgemäß, den Stamm Yarrowia lipolytica HR145 (DSM 12397) zu kultivieren.

Als Substrat für die erfindungsgemäß eingesetzte Kultur kommen synthetische, halbsynthetische oder komplexe Kulturmedien in Betracht. Diese enthalten kohlenstoffhaltige und stickstoffhaltige Verbindungen, anorganische Salze, gegebenenfalls Spurenelemente sowie Vitamine.

Als kohlenstoffhaltige Verbindungen kommen vorzugsweise Kohlenhydrate, Kohlenwasserstoffe oder organische Grundchemikalien in Betracht. Beispiele für vorzugsweise verwendbare Verbindungen sind Zucker, Alkohole bzw. Zuckeralkohole, organische Säuren oder komplexe Gemische. Bevorzugt sind erfindungsgemäß Öle.

Als Zucker kommt vorzugsweise Glucose in Betracht. Zu den verwendbaren Alkoholen gehören vorzugsweise Glycerin oder Mannit. Als organische Säuren kann vorzugsweise Zitronensäure zum Einsatz kommen. Zu den komplexen Gemischen zählen z.B. Malzextrakt, Hefeextrakt, Casein oder Caseinhydrolysat. Als Öl ist insbesondere Rizinusöl verwendbar. Hierbei können erfindungsgemäß auch Gemische zweier oder mehrerer der genannten Verbindungen eingesetzt werden.

Als stickstoffhaltige Substrate kommen anorganische Verbindungen in Betracht. Beispiele hierfür sind Nitrate und Ammoniumsalze. Ebenso können organische Stickstoffquellen zum Einsatz kommen. Hierzu zählen Hefeextrakt, Sojamehl, Baumwollsaatmehl, Casein, Caseinhydrolysat, Weizengluten und Maisquellwasser. Möglich ist auch der Einsatz zweier oder mehrerer der genannten Verbindungen als Gemisch.

Zu den einsetzbaren anorganischen Salze zählen beispielsweise Borate, Carbonate, Chloride, Molybdate, Nitrate, Phoshate und Sulfate. Als Metalle enthalten die genannten Salze vorzugsweise Calcium, Eisen, Kalium, Kobalt, Kupfer, Magnesium, Mangan, Natrium und Zink. Erfindungsgemäß kann auch ein Gemisch zweier oder mehrerer der genannten Salze verwendet werden.

Die Temperatur für die Kultivierung liegt vorzugsweise im Bereich von 10 bis 40°C. Besonders bevorzugt ist der Bereich von 20 bis 35°C, höchst bevorzugt sind 25 bis 30°C.

Der pH-Wert des Mediums beträgt bevorzugt 4 bis 9. Besonders bevorzugt ist der Bereich von 5 bis 8.

Während des Herstellungsverfahrens ist eine ausreichende Belüftung erforderlich. Entsprechend sind die erfindungsgemäß einsetzbaren Reaktoren auszulegen. Grundsätzlich können somit erfindungsgemäß alle dem Fachmann-bekannten Bioreaktoren eingesetzt werden, die für aerobe Verfahrensweisen geeignet sind. Vorzugsweise kommen alle für alle aerobe Submersverfahren geeigneten Vorrichtungen Betracht. Das heißt, es können erfindungsgemäß Gefäße ohne oder mit mechanischer Mischeinrichtung eingesetzt werden. Zu ersteren zählen z B. Schüttelapparaturen, Blasensäulen- oder Schlaufenreaktoren. Zu letzteren gehören vorzugsweise alle bekannten Vorrichtungen mit Rührem in beliebiger Gestaltung.

Das erfindungsgemäße Verfahren kann kontinuierlich oder diskontinuierlich durchgeführt werden. Die Dauer der Fermentation bis zum Erreichen einer maximalen Produktmenge liegt im Bereich von 36 und 72 Stunden, vorzugsweise im Bereich von 48 und 66 Stunden, gerechnet von dem Animpfen der Kultur.

Erfindungsgemäß können die Substrate zu Beginn der Inkubation, während oder nach Abschluß des Wachstums zugegeben werden. Dies kann mit einer einmaligen Zugabe an Substraten oder durch kontinuierliche, sukzessive Zugabe während des Prozesses erfolgen.

Bevorzugt ist jedoch die kontinuierliche Zugabe über einen Zeitraum von mehreren Stunden nach dem Animpfen der Kultur.

Mit den beschriebenen erfindungsgemäßen Verfahren ist es überraschenderweise möglich, in 65 Stunden mehr als 11 g/l zu erzeugen. Zu den folgenden Beispielen werden die Erfindung und die damit verbundenen Überraschungen näher erläutert.

### Beispiele

### 1. Herstellung der Vorkultur

1,7 g Malzextrakt werden in 100 ml Wasser in einem 500 ml Erlenmeyerkolben mit seitlichem Einstich gelöst und für 15 Minuten bei 121°C im Autoklaven sterilisiert. Nach dem Abkühlen auf Raumtemperatur wird der Malzbouillon-Kolben mit einer Impfschlinge von einer Schrägröhrchenkultur von Yarrowia lipolytica HR 145 angeimpft. Der Kolben wird für 24 Stunden auf der rotierenden Schüttelmaschine bei 27°C und 100 Upm inkubiert.

Zwei 500 ml Erlenmeyerkolben mit seitlichem Einstich werden mit Medium (1,461 g Na₂HPO₄ × 12 H₂O; 0,352 g KH₂PO₄; 0, 53 g Harnstoff; 0,07 g Tween 80; 5 g Hefepulver; 1 g Rizinusöl und 100 ml Wasser) beschickt und für 15 Minuten bei 121°C im Autoklaven sterilisiert. Nach Abkühlen auf Raumtemperatur werden die Kolben mit je 500 µl einer Malzextrakt-Bouillonkultur von Yarrowia lipolytica HR 145 angeimpft.

Die Kolben werden für 24 Stunden auf der rotierenden Schüttelmaschine bei 27°C und 100 Upm inkubiert.

### 2. Produktion von γ-Decalacton im 10 l Fermenter

Es werden 9,8 l Wasser im Fermenter vorgelegt und 14,61 g Na₂HPO₄ × 12 H₂O, 53 g Harnstoff, 50 g MgSO₄ × 7 H₂O, 0,04 g Riboflavin, 500 g Hefepulver, 7,0 g Tween 80, 100 g Rizinusöl und 5 g Antischaummittel zugefügt. Das Medium wird in situ 30 Min. bei 121°C sterilisiert. Weiterhin werden je 500 g verdünnte Natronlauge und Schwefelsäure sowie Rizinusöl im Autoklaven sterilisiert. Nach dem Abkühlen werden die Antischaumsonde und die Anstechgarnitur für NaOH angeschlossen. Der pH-Wert beträgt nach der Sterilisation etwa pH 7,9. Mittels der verdünnten Schwefelsäure wird ein pH-Wert von pH 7,0 eingestellt. Die Drehzahl der Rührung beträgt 400 Upm; die Belüftung 3 l/min Druckluft; die Temperatur 27°C.

Der Fermenter wird über eine sterile Anstechgarnitur mit der Vorkultur angeimpft. Während der Fermeritation wird weitere Natronlauge zudosiert, um den pH-Wert bei pH 7,0 zu halten. Bei Bedarf wird automatisch Entschäumer zudosiert. 14 Stunden nach dem Animpfen wird mit der Substratzugabe begonnen. Innerhalb von 4 Stunden werden 500 g Rizinusöl zugegeben.

Nach einer Fermentationszeit von ca. 52 Stunden wird die Fermentation beendet. Der Zeitpunkt für den Abbruch der Fermentation ist erreicht, wenn eine Stunde keine weitere Natronlauge zudosiert wurde. Um die Fermentation abzubrechen, wird der Fermentirinhalt mit konzentrierter Schwefelsäure auf pH 2,0 eingestellt und 30 Min. auf 80°C erhitzt. Nach dem Abkühlen wird er für die nachfolgende Aufarbeitung abgefüllt. Die Endkonzentration des γ-Decalactons beträgt laut HPLC 11.500 bis 12.500 ppm. Das Verhältnis 3-Hydroxy-γ-Decalacton zu γ-Decalacton ist kleiner 0,2.

Aus der so erhaltenen Kulturbrühe wird dann, gegebenenfalls nach Zentifugation, das γ-Decalacton mit den bekannten physikalischen Verfahren (Destillation, Extraktion, etc.) isoliert.

### 3. Produktion im 300 l Maßstab

Es wurden 200 ml Vorkultur genau nach Beispiel 1 hergestellt und zum Beimpfen eines 300 l Fermenters verwendet. Zuvor wurden 140 l Wasser im Fermenter vorgelegt und 742 g Harnstoff, 49 g KH₂PO_{4,}12 g H₂O, 7 g Hefeextrakt, sowie 98 g Tween 80 hinzugefügt. Das Medium wird in situ 30 Minuten bei 121°C sterilisiert.

Nach dem Abkühlen werden die Antischaumsonde und die Anstechgarnitur für NaOH angeschlossen. Der pH-Wert beträgt nach der Sterilisation etwa 7.0. Die Drehzahl des Rührers wird auf 180 Upm, die Belüftung auf 35 l/min und die Temperatur auf 27°C eingestellt. Der Fermenter wird unter sterilen Bedingungen mit den 200 ml Vorkultur angeimpft. Während der Fermentation wird mittels Natronlauge der pH-Wert auf pH 7.0 konstant gehalten. Etwa 16 Stunden nach dem Animpfen wird mit der Rizinusölzugabe (7.4 kg) begonnen, welche nach weiteren 4 Stunden abgeschlossen ist.

Nach einer Fermentationszeit von 65 Stunden wird die Fermentation beendet. Die Endkonzentration des γ-Decalactons beträgt 12 g/l.

## Patentansprüche

1. Verfahren zur Herstellung von γ-Decalacton in Bioreaktoren dadurch gekennzeichnet, daß Yarrowia lipolytica enthaltende Kulturen eingesetzt werden.

2. Verfahren nach Anspruch 1 dadurch gekennzeichnet, daß eine aus Yarrowia lipolytica bestehende Reinkultur eingesetzt wird.

3. Verfahren nach einem Ansprüche 1 oder 2 dadurch gekennzeichnet, daß Yarrowia lipolytica HR 145 (DSM 12397) eingesetzt wird.

4. Verfahren nach einem Ansprüche 1 bis 3 dadurch gekennzeichnet, daß synthetische, halbsynthetische oder komplexe Substrate eingesetzt werden.

5. Verfahren nach einem der Ansprüche 1 bis 4 dadurch gekennzeichnet, daß kohlenstoffhaltige sowie stickstoffhaltige Verbindungen, anorganische Salze, Spurenelemente und/oder Vitamine enthaltende Substrate eingesetzt werden.

6. Verfahren nach Anspruch 5 dadurch gekennzeichnet, daß das Substrat als kohlenstoffhaltige Verbindungen Zucker, Zuckeralkohole, Alkohol, organische Säuren, komplexe Gemische, Öle oder Gemische zweier oder mehrerer dieser Substanzen enthält.

7. Verfahren nach Anspruch 6 dadurch gekennzeichnet, daß Glucose, Glyzerin, Mannit, Zitronensäure, Malzextrakt, Hefeextrakt, Casein, Caseinhydrolysat und Rizinusöl oder Gemische zweier oder mehrerer dieser Substanzen enthaltende Substrate eingesetzt werden.

8. Verfahren nach einem der Ansprüche 1 bis 7 dadurch gekennzeichnet, daß anorganische Verbindungen und/oder organische Verbindungen enthaltenden Substrate eingesetzt werden.

9. Verfahren nach Anspruch 8 dadurch gekennzeichnet, daß Nitrate, Ammoniumsalze, Hefeextrakt, Sojamehl, Baumwollsaatmehl, Casein, Caseinhydrolysat, Weizengluten und Maisquellwasser eingesetzt werden.

10. Verfahren nach einem der Ansprüche 1 bis 9 dadurch gekennzeichnet, Sulfate, Nitrate, Chloride, Carbonate und Phosphate der Metalle Natrium, Kalium, Magnesium, Mangan, Calcium, Zink und Eisen oder Gemische zweier oder mehrerer dieser Verbindungen enthaltende Substrate eingesetzt werden.

11. Verfahren nach einem der Ansprüche 1 bis 10 dadurch gekennzeichnet, daß die Temperaturen im Bereich von 10 bis 40°C liegen, vorzugsweise bei 20 bis 35°C.

12. Verfahren nach einem der Ansprüche 1 bis 11 dadurch gekennzeichnet, daß der pH-Wert im Bereich von 4 bis 11, vorzugsweise 5 bis 8 liegt.

13. Yarrowia lipolytica HR 145 (DSM 12397).

14. Verwendung von Yarrowia lipolytica nach Anspruch 13 zur Herstellung von γ-Decalacton.
